# EUROPEAN PATENT APPLICATION

(11) **EP 2 517 567 A1**
(43) Date of publication of application: **31.10.2012**
(21) Application number: 09852474.7
(22) Date of filing: 23.12.2009
(51) Int. Cl.: A23G 1/44, A23J 3/34

(54) **OBTAINMENT OF BIOACTIVE PRODUCTS FROM COCOA HAVING INHIBITORY ACTIVITY AGAINST THE PEP ENZYME AND ANTIOXIDANT AND/OR ANTINEURODEGENERATIVE ACTIVITY**

(71) Applicant: Biopolis S.L., 46980 Paterna (Valencia) (ES); Natraceutical Industrial S.L.U., 46930 Quart De Poblet, Valencia (ES)
(72) Inventor: BATALLER LEIVA, Esther, 46980 Valterna-Paterna (Valencia) (ES); GENOVÉS MARTINEZ, Slavador, 46960 Aldaia (Valencia) (ES); MARTORELL GUEROLA, Patricia, 46220 Picassent (Valencia) (ES); RAMON VIDAL, Daniel, 46183 L'Eliana (Valencia) (ES); IBAÑEZ LOPEZ, Aida, 46181 Benisanó (Valencia) (ES); LLOPIS PLA, Silvia, 46839 Guadassequies (Valencia) (ES); GONZALEZ MARTINEZ, Nuria, 46970 Alacuas (Valencia) (ES); MONZO OLTRA, Honorato, E-46182 La Canyada-Paterna (Valencia) (ES); MUGUERZA MARQUINEZ, Begoña, 46015 (Valencia) (ES)
(74) Representative: Gonzalez-Alberto Rodriguez, Natalia
(86) International application number: PCT/ES2009/000597
(87) International publication number: WO 2011/076954

(57) **Abstract**

The present invention relates to the production of bioactive products from raw plant matter, namely cocoa extracts. The said products have one or more biopeptides with prolyl endopeptidase (PEP) enzyme inhibitory activity *in vitro* and/or antioxidant and/or antineurodegenerative capacity *in vivo* and can be used in dietetics and in the food and pharmaceutical industries.

## Description

### TECHNICAL FIELD OF THE INVENTION

The present invention relates to the production of bioactive products from raw plant matter, namely cocoa extracts. The said products have one or more biopeptides with prolyl endopeptidase (PEP) enzyme inhibitory activity *in vitro* and/or antioxidant and/or antineurodegenerative capacity *in vivo* and can be used in dietetics and in the food and pharmaceutical industries.

### BACKGROUND OF THE INVENTION

This application can be considered a continuation application of patent application PCT/ES2008/000540, by the same authors of the present invention, wherein bioactive products obtained by enzymatic hydrolysis are described, comprising one or more of a series of eight peptides that have been isolated and characterised therein by their sequences. The sequences of these eight peptides are the following: SDNEWAWMF (SEQ.ID. No. 29); LSDNEWAWMF (SEQ. ID. No. 30); SDNEWAWMFK (SEQ. ID. No. 31); LSDNEWAWMFK (SEQ. ID. No. 32); RRSDLDNGTPVIF (SEQ. ID. No. 33); DNYDNSAGKWWVT (SEQ. ID. No. 34); TSTVWRLDNYDNSA (SEQ. ID. No. 35) and DNYDNSAGKWWVTTD (SEQ. ID. No. 36).

The proteins in foods are precursors of a large number of peptides with special biological activity, having useful properties affecting different bodily processes. Due to the great interest in developing natural products that provide some sort of "extra" beneficial health effect for persons consuming them, there is a good opportunity to use various raw plant matter sources to develop new products and functional ingredients. The possibility of generating these peptides is a state-of-the-art research field in the nutraceutical industry as it enables the generation of new functional food applications, giving rise to the added value of food components and byproducts, improving the nutritive properties of conventional foods and developing new dietary supplements or even new medicinal products.

Some byproducts of the agro-food industry have a high content of proteins and bioactive peptides, conferring added value to the same. There are different mechanisms to enhance increased peptide content by considerably enriching a matrix and conferring to it specific characteristics, such as for example, antihypertensive, antihyperglycemic, antineurodegenerative, anticariogenic, and even antihyperlipidemic characteristics.

Proteolytic enzymes can be used to hydrolyze proteins at specific points, capable of generating a broad range of peptides in the hydrolyzed products obtained with multiple physiological effects.

References to the state of the art can be found in the following documents, among others: the patent application WO91/19800 A1, which relates to proteins and nucleic acids derived from cocoa. Specifically, claims relate to fragments of the protein albumin (21kDa), from the cacao seed, as cocoa aroma precursors. Said patent application assigns to the peptide of 205 amino acids, or fragments of the same, the functionality of being precursors of cocoa flavour.

European patent EP1298210 A1 describes peptides derived from albumin and vicilin proteins derived from the cocoa bean as chocolate flavour precursors. The authors of the aforementioned document attribute to the peptides, of between 2 and 30 amino acids, preferably between 2 and 5 amino acids, the functionality of being precursors of chocolate flavour. Likewise, said authors also claim the use of these peptides for the manufacture of ice-cream, beverages, dairy products, cosmetics, animal feed and pharmaceuticals.

Patent application WO96/38472 A1 claims the peptides that are cocoa flavour precursors to be the peptides with the amino acid sequence Lys-Ala-Pro-Leu-Ser-Pro-Gly-Asp-Val-Phe-Val and fragments from 2 to 11 amino acids contained in the said sequence. The claimed sequence and fragments thereof are attributed the functionality of being cocoa flavour precursors.

Patent application WO02/42327 A2 relates to the process of isolation, purification and identification of 2S albumin protein, as cocoa flavour precursor protein. Specifically, said application describes the use of the said polypeptide or fragments thereof for the production of cocoa/chocolate flavour.

Patent document JP2008019228 describes an amino acid composition derived from cocoa extracts subjected to prior extraction of polyphenols with angiotensin-1 converting enzyme (ACE) inhibitory properties, as well as the foods and foodstuffs containing the said composition. The said document does not specify the peptide/amino acid sequence comprised by the said extract nor does it mention the isolation and/or purification process employed.

Prolyl-endopeptidase (PEP) enzyme activity is associated with loss of memory and learning processes because it degrades the proline-rich neuropeptides, such as vasopressin and substance P, involved in these processes. Some studies also indicate that this enzyme could be linked to Alzheimer's disease. To date, several authors such as Kim et al., ("Prolyl Endopeptidase Inhibitors from Green Tea", Arch Pharm Res, 24 (4) :292-296 2001) and Tezuka et al. ("Screening of crude drug extracts for prolylendopeptidase inhibitory activity", Phytomedicine, 6(3) :197-203 1999), assert that the polyphenol extracts of certain plants cause prolyl endopeptidase inhibition. Patent document US 2007/0116779 also describes the use of cocoa beans as a polyphenol source, as one of the elements of pharmaceutical compositions and as an inhibitor of certain enzymes involved in neurodegenerative diseases, such as Alzheimer's or Parkinson's.

Other authors, such as Maruyama et al., ("Prolyl Endopeptidase Inhibitory Activity of Peptides in the Repeated Sequence of various Proline-Rich proteins", Journal of Fermentation and Bioengineering, 74:145-148 1992) and Asano et al., ("Inhibition of prolyl endopeptidase by synthetic peptide fragments of human beta-casein"; Agric.Biol. Chem, 55(3):825-828 1991) state that some peptide fragments inhibit PEP activity; however, to date there are no references to cocoa peptides as inhibitory metabolites of the said enzyme.

Therefore, due to the increasing demand for new ingredients and compounds with this kind of inhibitory activity, given the healthy characteristics of cocoa, the possibility was considered of finding bioactive peptides from cocoa extracts that exert PEP inhibitory activity. Eight peptides were isolated that not only have PEP inhibitory activity *in vitro,* but said bioactive peptides also show antioxidant and/or antineurodegenerative capacity *in vivo* according to assays with the nematode model *C. elegans,* which gave rise to the patent application PCT/ES/000540.

Ongoing research has surprisingly found new bioactive products comprising 24 new peptides.

### OBJECT OF THE INVENTION

The present invention relates to the production of bioactive products from cocoa extracts rich in new bioactive peptides with PEP enzyme inhibitory activity *in vitro* and/or antioxidant and/or antineurodegenerative capacity *in vivo.*

Specifically, the invention relates to obtaining purified peptide fractions from cocoa that have PEP enzyme inhibitory activity and/or antioxidant and/or antineurodegenerative capacity for potential use as ingredients to be incorporated in functional foods.

The bioactive peptides can be produced by hydrolysis of one or more proteins or peptides present in the cocoa extracts obtained. For this purpose, enzymes and hydrolysis conditions are set to obtain the desired biopeptides.

Different methodologies may be used to study these inhibitory activities. *In vitro* tests are based on the determination of PEP enzyme enzymatic activity in the presence of the inhibitor in question, i.e., in the present invention this relates to the peptide-rich cocoa fractions.

The bioactive peptides or the hydrolysates containing them can be incorporated into functional foods. Once concentrated, the fractions can be used in the food industry and in dietary products as well as in the pharmaceutical industry for medicinal product manufacture. The said bioactive products containing a suitable amount of the said peptides can be used in the treatment and prevention of diseases, such as blood pressure control, namely hypertension, and also degenerative-type diseases such as Alzheimer's, Parkinson's, etc. They can also be used as antioxidants. Thus, the invention finds new applications for cocoa, contributing to its positive reassessment from the health viewpoint.

The 24 peptides, object of this invention, that the bioactive products may contain which, on having been sequenced and identified, can be obtained from any raw material source or substrate that contains them. In addition, the new bioactive peptides identified in the hydrolysates can be obtained by chemical and/or enzymatic peptide synthesis or by recombinant methods.

### BRIEF DESCRIPTION OF THE DRAWINGS

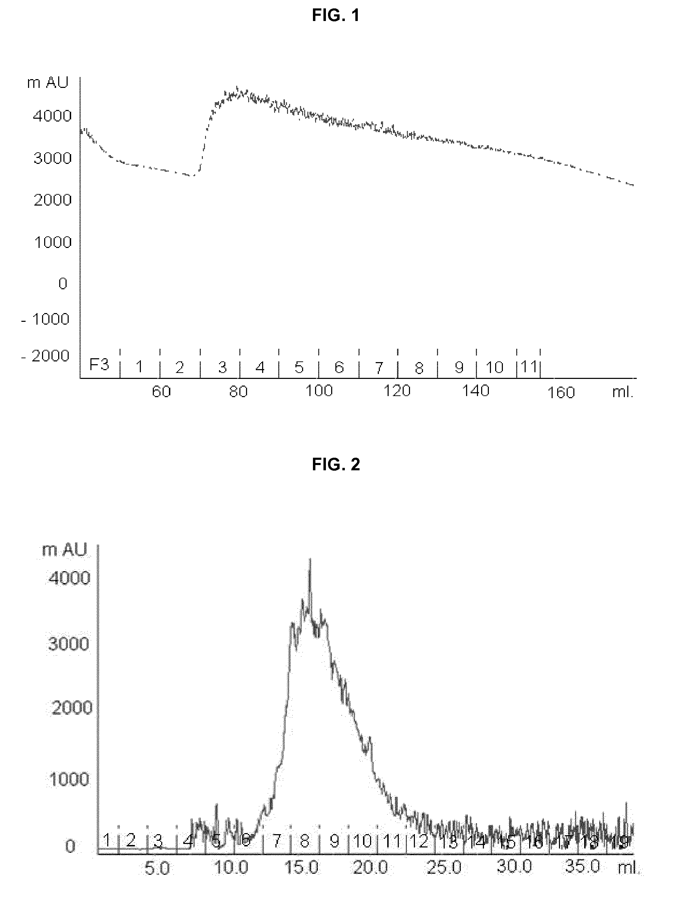

**Figure 1**. Hydrophobic interaction chromatograph (HIC). On observing the curve at 214 nm, it is concluded that all the protein is contained in fractions 3 and 4.
**Figure 2**. Reversed-phase chromatograph of HIC (hydrophobic interaction chromatography) fraction 3. On observing the curve at 214 nm, it can be concluded that all the protein is contained in fractions 7 and 11.

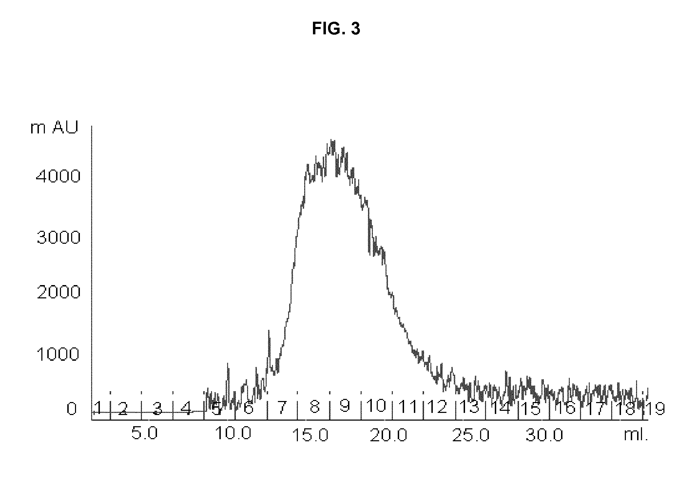

**Figure 3**. Reversed-phase chromatograph of HIC fraction 4. On observing the curve at 214 nm, it can be concluded that all the protein is contained in fractions 7 and 11.

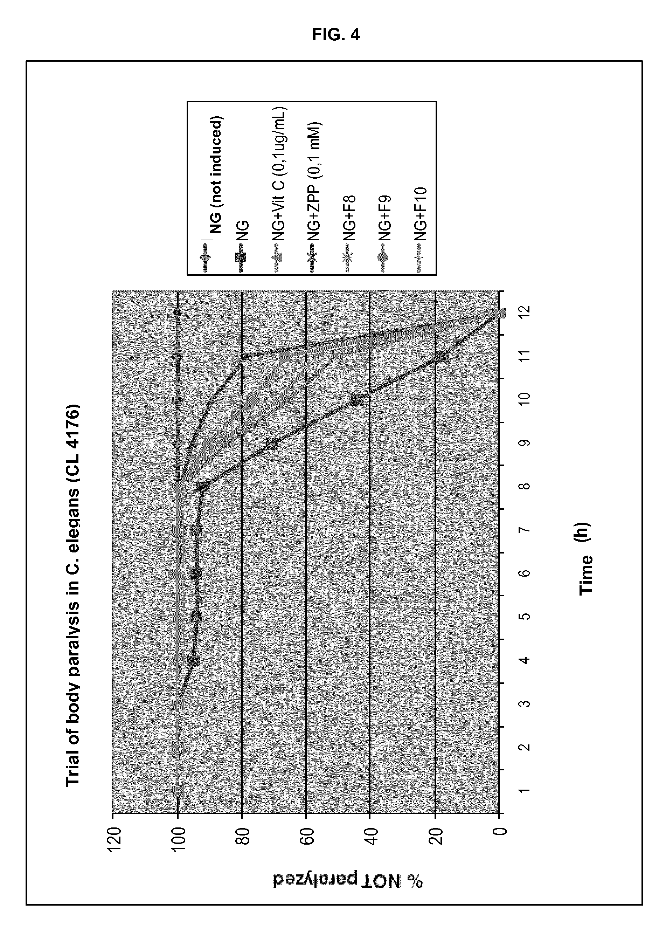

**Figure 4**. Assay of the protective capacity against the accumulation of amyloid-beta peptide in C. *elegans* CL4176 with fractions F8 (31.3 µg protein/ml); F9 (18.1 µg protein /ml) and F10 (9.9 µg protein/ml). Positive controls were ZPP (0.1 mM) and vitamin C (0.1 mg/ml). From this figure it can be concluded that fractions F8, F9 and F10 provide protection against the accumulation of B-amyloid petide, with F9 and F10 being the fractions conferring the greatest protection.

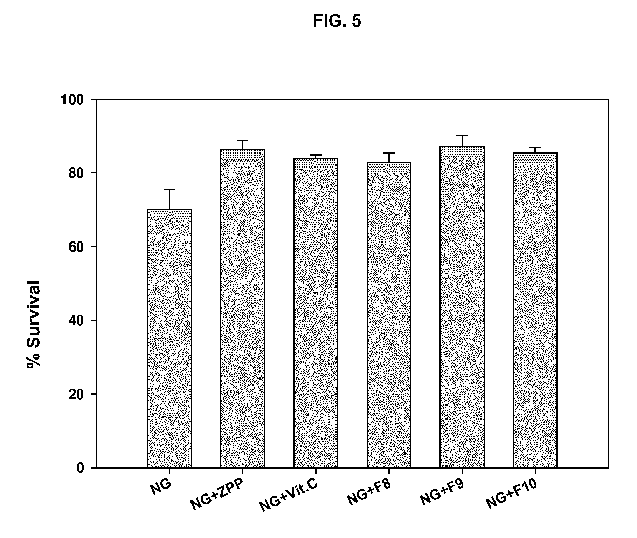

**Figure 5****.** Effect of peptide extracts F8 (31.3 µg protein/ml); F9 (18.1 µg protein/ml) and F10 (9.9 µg protein/ml) on survival in *C*. *elegans* CL2070 strain. Positive controls were ZPP (0.1 mM) and vitamin C (0.1 mg/ml). The bars indicate standard deviations. The figure shows how when subjected to oxidative stress conditions, there was an increase in survival rates of nematodes treated with peptide extracts from fractions F8, F9 and F10.

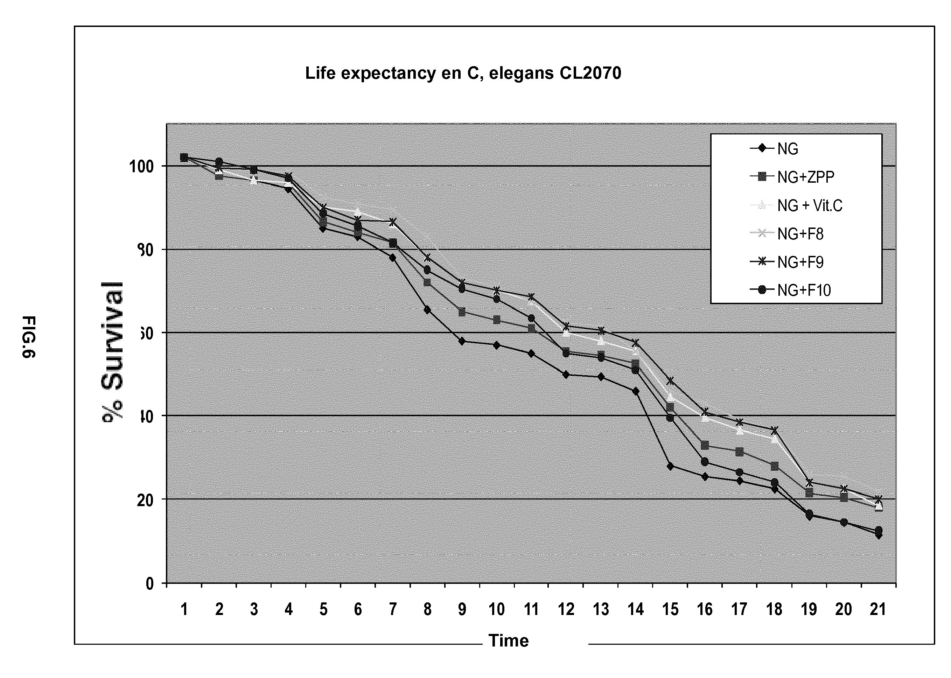

**Figure 6****.** Effect of peptide extracts F8 (31.3 µg protein/ml), F9 (18.1 µg protein/ml) and F10 (9.9 µg protein/ml) on the survival of the nematodes of the *C.elegans* CL2070 strain. ZPP (0.1 mM) and vitamin C (0.1 mg/ml) were used as positive controls. This figure shows the increased survival curve corresponding to nematodes treated with peptide extracts from fractions F8, F9 and F10.

### DETAILED DESCRIPTION OF THE INVENTION

The present invention relates to a method to obtain bioactive products, specifically from fractions of cocoa rich in new peptides with PEP inhibitory activity *in vitro* and/or antioxidant and/or antineurodegenerative capacity *in vivo.* Specifically, the invention is based on obtaining different peptide fractions by enzymatic hydrolysis of a cocoa byproduct (*bark*) to obtain bioactive peptides, with the purpose of their potential use as ingredients for incorporation into functional foods

Another aspect of the invention relates to purification of the peptides from the cocoa extract obtained. Different purification strategies are used depending on the peptides present in the extract, for example, concentration by ion-exchange or hydrophobic interaction, and separation, for example by RPC (reverse phase chromatography) or gel filtration chromatography.

The present invention relates to 24 new bioactive peptides from cocoa extracts. The said bioactive peptides are identified with the amino acid sequences denoted: SEQ. ID. N°. 1; SEQ. ID. N°. 2; SEQ. ID. N°. 3; SEQ. ID. N°. 4; SEQ. ID. N°. 5; SEQ. ID. N°. 6; SEQ. ID. N°. 7; SEQ. ID. N°. 8;; SEQ. ID. N°. 10;; SEQ. ID. N°. 12; SEQ. ID. N°. 13; SEQ. ID. N°. 14; SEQ. ID. N°. 15; SEQ. ID. N°. 16; SEQ. ID. N°. 17; SEQ. ID. N°. 19; SEQ. ID. N°. 20; SEQ. ID. N°. 22; SEQ. ID. N°. 23; SEQ. ID. N°. 24; SEQ. ID. N°. 25; SEQ. ID. N°. 26; SEQ. ID. N°. 27 and SEQ. ID. N°. 28, which have PEP enzyme inhibitory activity *in vitro* enzyme and/or antioxidant and/or antineurodegenerative capacity *in vivo.*

Furthermore, the invention relates to the use of the said bioactive peptides as functional ingredients for different foods, considering foods to be any composition intended for consumption; be it liquid or solid.

The raw material source of the present invention may be any appropriate substrate comprising one or more of the bioactive peptides described herein. In a particular embodiment, the said raw material is a plant, namely the raw material source is cocoa. *Theobroma cacao* is the scientific name of the cacao tree or cocoa tree. The term cocoa comes from Mayan (*Ka'kaw*) and *Theobroma* is Greek for "food of the gods". More specifically a cocoa byproduct called *bark* is used, which is cocoa with the husk partially defatted by means of physical pressing, with a fat proportion that can range between 5% and 15%, preferably 10%, expressed in weight percentage.

The said raw material source is dissolved in water at a rate that can vary between 5% and 20%; preferably 10%. Once the *bark* is dissolved in water it is treated at different temperatures; for example between 40 °C and 60 °C, preferably 50 °C for different time periods; for example between 1 and 24 hours; preferably 1, 6, 18 or 24 hours with one or more hydrolytic enzymes at different concentrations. Any enzyme capable of providing the peptides of interest can be used. Specifically, the enzymes used are equally enzymes with cellulose activity, as is the case of the enzyme Termamyl, and enzymes with protease activity, such as the enzymes Alcalase, Neutrase, Ultraflo or Flavourzyme. A single enzyme or combinations of two or more enzymes can be used, provided that they produce one or more biopeptides with the desired characteristics. The enzyme concentrations used range between 0.10 and 10 µl/L, and between 0.5 and 2 µl of enzyme per g of raw material. The hydrolysis conditions are: pH, temperature, pressure, concentration of enzyme(s), reaction time, etc. are optimized depending on the enzyme or enzymes used.

To obtain the extract, once the hydrolysis reaction has ended, the enzyme/enzymes is/are inactivated. Centrifugation at 4000 rpm is performed for 15 minutes and the supernatant containing the bioactive peptides, object of the present invention, is collected and then separated into fractions and subfractions for isolation and purification.

Therefore, another aspect of the invention relates to the purification and identification of the bioactive peptides obtained from the cocoa extracts. Given that the peptides present in the extract are of a different amino acid length and considering that the bioactive peptides of interest have between 5 and 20 amino acids, different purification strategies can be carried out both for removing unwanted peptides and for isolating and concentrating the peptides object of the invention. For example, fractions of a different molecular weight can be obtained from the hydrolyzed products by means of ultrafiltration, with subsequent active subfraction isolation by means of ion exchange or hydrophobic interaction or high performance reverse phase or gel filtration chromatography.

The advantages of the present invention are not only due to the new peptides previously referred to as SEQ. ID. No. 1; SEQ. ID. No. 2; SEQ. ID. No. 3; SEQ. ID. No. 4; SEQ. ID. No. 5; SEQ. ID. No. 6; SEQ. ID. No. 7; SEQ. ID. No. 8; SEQ. ID. No. 10; SEQ. ID. No. 12; SEQ. ID. No. 13; SEQ. ID. No. 14; SEQ. ID. No. 15; SEQ. ID. No. 16; SEQ. ID. No. 17; SEQ. ID. No. 19; SEQ. ID. No. 20; SEQ. ID. No. 22; SEQ. ID. No. 23; SEQ. ID. No. 24; SEQ. ID. No. 25; SEQ. ID. No. 26; SEQ. ID. No. 27 and SEQ. ID. No. 28; but also the raw material from which the said peptides can be obtained or are initially present in the whole hydrolyzed products and fractions obtained from the initial cocoa having bioactive properties, simultaneously showing PEP enzyme inhibitory activity, and/or also antioxidant and/or antineurodegenerative activity. Both the peptides and the extracts containing them can be incorporated in functional foods as well as forming part of pharmaceutical or dietetic compounds, and be used to help in the treatment and prevention of different diseases, especially cardiovascular disease and cerebral degeneration.

### EXAMPLES

### Example 1: Preparation of the cocoa extract, rich in bioactive peptides. The Forastero cocoa variety from the Ivory Coast was used to carry out the process.

The process of obtaining the *bark* was performed with fresh cacao seeds obtained from cocoa pods by the process according to the patent document ES 2286947 A1 and PCT/ES2008/000540, by the same authors of the present invention. The product obtained after defatting by pressing is referred to as *bark* if it is from beans with the husk, as in the case of the present invention. To that end, 100 g of *bark,* which had been defatted by pressing in a continuous mechanical extractor, and had a residual fat content of 10%, was dissolved in 1 L of distilled water at a 1:10 (w:v) ratio with a combination of Termamyl and Alcalase enzymes, at a concentration of 1 µl/g of each of them and maintained with stirring for 1 hour at 50 °C. After this time had elapsed, enzymes were inactivated at 100 °C and centrifugation was performed at 4000 rpm for 15 minutes and approximately 850 ml of peptide-rich supernatant was recovered. This supernatant was used to carry out bioactive peptide purification and PEP enzyme inhibition assay.

### Example 2: Purification of peptides from cocoa extract. The supernatant containing the peptides and obtained after centrifugation was subjected to two purification steps.

The first step of the process consisted of subjecting the samples to a concentration phase by means of hydrophobic interaction chromatography (HIC) in a ÄKTA Explorer chromatographer (GE Healthcare, Amersham Biosciences AB). To that end the HiPrep 16/10 Phenyl FF (high sub) column was used and the balancing buffer (100 mM sodium phosphate, 1.5 M (NH₄)₂SO₄, pH=7) with a gradient of 1.5 at 0 M of (NH₄)₂SO₄ was used for the subsequent elution. The said elution was monitored at 214 nm and PEP inhibitory activity was evaluated *in vitro* in each of the 10 ml fractions obtained.

The fractions having activity were subjected to a purification step by means of a first ultrafiltration step using 10 kDa filters (Amicon Ultra, Millipore), and subsequent reverse phase chromatography (RPC) of the fraction below 10 kDa, using the RESOURCE RPC 3 ml column (Amersham Biosciences) in an ÄKTA Explorer chromatographer (Amersham Biosciences) and using a broad elution gradient with eluents 0.1% TFA in miliQ water (A) and 0.1% TFA in acetonitrile (B). The samples were monitored at 214 nm and, after removing the solvents used in the process, PEP inhibitory activity was re-evaluated *in vitro* in each of the fractions obtained, which were of 2 ml in this case.

### Example 3. Evaluation of PEP enzyme inhibitory activity in vitro.

### A. - Evaluation of PEP inhibitory activity.

Prolyl endopeptidase (PEP) enzyme activity was measured according to the method described by Yoshimoto (Yoshimoto, T. and Tsuru, D. 1978. Agr. Biol. Chem., 42, 2417; Yoshimoto, T., Walter, R. and Tsuru, D. 1980. J. Biol. Chem., 255, 4786), based on the use of substrate Z-Gly-Pro-*p*-nitroaniline, from which p-nitroaniline is released due to the action of the PEP enzyme, which can be spectrophotometrically quantified by taking absorbance readings at 410 nm.

PEP inhibitory activity was determined by adding the test sample to the reaction mixture. The initial *bark* inhibition was determined as control without enzymatic treatment, as well as after protein hydrolysis with commercial enzymes and the purification HIC fractions (Figure 1).

Different fractions were separated by hydrophobic interaction chromatography (HIC). Table 1 shows the PEP inhibition percentage of the bark samples and of the different fractions.
Table 1 contains the results of PEP enzyme inhibition of all the samples throughout the first step of purification.

**Table 1. PEP inhibition percentage of bark samples as well as the hydrophobic interaction fractions.**

| **Sample** | **PEP inhibition** |
|---|---|
| Untreated bark | 22.81 ± 6.45 |
| Alcalase+Termamyl bark | 42.81 ± 2.98 |
| Filtered bark (150 ml) | 27.02 ± 1.49 |
| Non retained HIC | 14.39 ± 1.49 |
| F2 HIC | 3.86 ± 1.47 |
| F3 HIC | 18.95 ± 8.93 |
| F4 HIC | 35.44 ± 17.37 |
| F5 HIC | 1.40 ± 0.98 |
| F6 HIC | 1.40 ± 0.91 |
| F7 HIC | 0 .0 ± 0.0 |

Fractions 3 and 4, obtained subsequent to purification by hydrophobic interaction chromatography, were selected as having the greatest PEP enzyme inhibitory ability. Accordingly, they were subjected to reverse phase chromatography (Figures 2 and 3).
Tables 2 and 3 contain the PEP enzyme inhibition values of the fractions obtained by reversed-phase chromatography from the hydrophobic interaction fractions 3 and 4, respectively, as well as the protein content of each.

**Table 2. Percentage of PEP inhibition and protein quantity of the RPC fractions obtained from hydrophobic interaction fraction 3.**

| **Samples** | **Protein content (mg)** | **% PEP inhibition** |
|---|---|---|
| F6 RPC. (F3 HIC) | 0.041168081 | 10.18 ± 0.85 |
| F7 RPC. (F3 HIC) | 0.520117841 | 14.37 ± 3.39 |
| F8 RPC. (F3 HIC) | 5.329239984 | 24.55 ± 2.54 |
| F9 RPC. (F3 HIC) | 2.5620785 | 22.16 ± 0.85 |
| F10 RPC. (F3 HIC) | 1.131518808 | 19.16 ± 0.00 |
| F11 RPC (F3 HIC) | 0.470186129 | 8.38 ± 3.39 |
| F12 RPC. (F3 HIC) | 0.143528 | 10.78 ± 3.39 |
| F13 RPC. (F3 HIC) | 0.058311881 | 6.59 ± 7.62 |
| F20 RPC. (F3 HIC) | 0.039742304 | 8.38 ± 5.08 |

**Table 3. Percentage of PEP inhibition and protein quantity of the RPC fractions from hydrophobic interaction fraction 4.**

| **Sample** | **Protein content (mg)** | **% PEP inhibition** |
|---|---|---|
| F6 RPC. (F4 HIC) | 0.013445155 | 11.65 ± 5.11 |
| F7 RPC. (F4 HIC) | 0.393839581 | 20.48 ± 0.57 |
| F8 RPC. (F4 HIC) | 6.269268373 | 34.54 ± 2.27 |
| F9 RPC. (F4 HIC) | 3.61016316 | 38.96 ± 1.70 |
| F10 RPC. (F4 HIC) | 1.97302672 | 26.51 ± 3.41 |
| F11 RPC. (F4 HIC) | 0.889492386 | 22.49 ± 1.14 |
| F12 RPC. (F4 HIC) | 0.299741375 | 19.28 ± 0.00 |
| F13 RPC. (F4 HIC) | 0.100727615 | 13.25 ± 3.98 |

Since RPC fractions 8, 9 and 10 from the HIC fraction 4 showed the highest PEP enzyme inhibition values, they were selected for the evaluation of peptide content therein. The said fractions were analyzed by mass spectrometry MS / MS and 24 new peptides were identified, whose sequences are listed in Table 4.

**Table 4. Sequences of the 28 peptides identified in RPC fractions 8, 9 and 10, from HIC fraction 4 (peptides 8 and 12 were identified in both fractions 9 and 10).**

| | | **MW (Da)** | **Peptide sequence** |
|---|---|---|---|
| Fraction 8 FI (F4HIC) | SEQ.ID. No. 01 | 1067.37 | GVKGEPGPNTL |
| | SEQ.ID. No. 02 | 1173.45 | ALPVNSPGKYE |
| | SEQ.ID. No. 03 | 1283.41 | TDGVKGEPGPNTL |
| | SEQ.ID. No. 04 | 1292.45 | LSQSPVYSNQNG |
| | SEQ.ID. No. 05 | 1483.69 | VTTDGVKGEPGPNTL |
| Fraction 9 FI (F4HIC) | SEQ.ID. No. 06 | 1017.33 | SDDDGQIRL |
| | SEQ.ID. No. 07 | 1053.31 | NYDNSAGKW |
| | SEQ.ID. No. 08 | 1168.41 | DNYDNSAGKW |
| | SEQ.ID. No. 03 | 1283.53 | TDGVKGEPGPNTL |
| | SEQ.ID. No. 10 | 1437.47 | RLDNYDNSAGKW |
| | SEQ.ID. No. 05 | 1483.67 | VTTDGVKGEPGPNTL |
| | SEQ.ID. No. 12 | 1857.83 | ANSPVLDTDGDELQTGVQ |
| Fraction 10 FI (F4HIC) | SEQ.ID. No. 13 | 777.27 | GHAVTFF |
| | SEQ.ID. No. 14 | 904.31 | FASKDQPL |
| | SEQ.ID. No. 15 | 954.31 | TFGEFQQV |
| | SEQ.ID. No. 16 | 968.43 | VAPAGHAVTF |
| | SEQ.ID. No. 17 | 1029.35 | KAPLSPGDVF |
| | SEQ.ID. No. 07 | 1053.35 | NYDNSAGKW |
| | SEQ.ID. No. 08 | 1168.35 | DNYDNSAGKW |
| | SEQ.ID. No. 19 | 1239,53 | APLSPGDVFVAPA |
| | SEQ.ID. No. 20 | 1256,59 | QVKAPLSPGDVF |
| | SEQ.ID. No. 03 | 1283,57 | TDGVKGEPGPNTL |
| | SEQ.ID. No. 22 | 1367,73 | KAPLSPGDVFVAPA |
| | SEQ.ID. No. 23 | 1384,61 | QQVKAPLSPGDVF |
| | SEQ.ID. No. 24 | 1594,93 | QVKAPLSPGDVFVAPA |
| | SEQ.ID. No. 25 | 1629,81 | SQSPVYSNQNGRFF |
| | SEQ.ID. No. 26 | 1669,41 | WVTTDGVKGEPGPNTL |
| | SEQ.ID. No. 27 | 1758,79 | SQSPVYSNQNGRFFE |
| | SEQ.ID. No. 12 | 1857,83 | ANSPVLDTDGDELQTGVQ |
| | SEQ.ID. No. 28 | 2020,93 | ANSPVLDTDGDELQTGVQY |

### Example 4. In vivo evaluation of RPC (reverse phase chromatography) fractions.

### A. Evaluation of the antineurodegenerative capacity of the fractions in C.elegans.

The aim of this study was to evaluate *in vivo* the functionality of the RPC fractions that gave positive results for prolyl endopeptidase inhibition *in vitro.* With this purpose the model organism *Caenorhabditis elegans* was used. The said functionality is related to the potential protection provided by the said extracts against developing Alzheimer's neurodegenerative disease. To evaluate this capacity, RPC fractions 8, 9 and 10 from HIC fraction 4 were selected and a test was performed to determine body paralysis using *C*. *elegans* CL4176 transgenic strain. This strain is characterised in that it expresses the human amyloid-beta peptide (Ab1-42) after temperature induction.

Previously published data in the literature indicate that the formation of amyloid beta-peptide plaques is preceded by oxidative stress (Drake et al., "Oxidative stress precedes fibrillar deposition of Alzheimer's disease amyloid beta-peptide (1-42) in a transgenic Caenorhabditis elegans model" Neurobiol Aging 2003, 24(3):415-20). Therefore it is interesting to evaluate whether the effect exerted by the action of a molecule or compound provides greater resistance to the accumulation in fibrillar A-beta peptide deposits in the neurons. In these transgenic nematodes the expression of the said peptide causes bodily paralysis that can be evaluated after adding the molecule or compound of interest.

To conduct the experiment, age synchronized CL4176 strain nematodes were obtained by plate cultivation on NGM (nematode growth medium) at 16 °C. Eggs were collected on NGM plates containing 100 µL of each peptide fraction (F8, F9 and F10). Positive controls were Z-prolyl prolinal (100 µL of a 10 mM stock on the plate surface) at a final concentration of 0.1 mM; and vitamin C (25 µL of a 0.04 mg/ml stock) at a final concentration of 0.1 µg/ml. Negative controls were NGM and NGM-non induced (nematodes were incubated at 16 °C throughout the experiment). One hundred nematodes were analysed per condition. After 1 day of incubation (when nematodes were approximately 23 hours old), temperature was increased from 16 °C to 25 °C to induce Ab peptide expression. Then 24 hours after induction, temperature was dropped to 20 °C and maintained for the rest of the trial. After induction, the number of paralyzed nematodes was analyzed for each condition tested until the percentage of paralyzed nematodes reached 100%.

Figure 4 shows the results obtained on completion of the body paralysis trial using *C. elegans* CL4176 strain. It is evident that the fractions studied (F8, F9 and F10) provide protection against amyloid-beta peptide accumulation compared to the NGM-induced control, with fractions F9 and F10 conferring the greatest protection, at similar levels. The nematodes not induced with amyloid-beta peptide do not suffer paralysis.

### B. Evaluation of the antioxidant capacity of the peptide fractions in C.elegans.

To conduct the experiment, age synchronized CL2070 strain nematodes were obtained by plate cultivation on NGM at 20 °C. Eggs were collected on NGM plates containing 100 µL of each peptide fraction (F8, F9 and F10). Positive controls were Z-prolyl prolinal (100 µL of a 10 mM stock on the plate surface) at a final concentration of 0.1 mM; and vitamin C (25 µL of a 0.04 mg/ml stock) at a final concentration of 0.1 µg/ml. Negative control was NGM. One hundred nematodes were assayed per condition. After 7 days of incubation under the said conditions, the nematodes were subjected to oxidative stress. To this end, nematodes were transferred to plates with S basal medium containing H₂O₂ (2 mM). The plates were incubated at 20 °C and after 5 hours the total number of nematodes surviving the treatment was recorded.

Figure 5 shows the increase in survival of nematodes treated with peptide extracts F8, F9 and F10 after being subjected to oxidative stress with H₂O₂ (2 mM) for 5 hours, as compared to the negative NGM control.

### C. Evaluation of C.elegans survival after addition of the peptide fractions to the culture medium.

To conduct the experiment, age synchronized CL2070 strain nematodes were obtained by plate cultivation on NGM at 20 °C. Eggs were collected on NGM plates containing 100 µL of each peptide fraction (F8, F9 and F10). Positive controls were Z-prolyl prolinal (100 µL of a 10 mM stock on the plate surface) at a final concentration of 0.1 mM; and vitamin C (25 µL of a 0.04 mg/ml stock) at a final concentration of 0.1 µg/ml. Negative control was NGM. One hundred nematodes were assayed per condition. Every 2 days the number of nematodes surviving in each condition was counted until there were practically no living nematodes left.

Figure 6 shows the survival curve for nematodes grown in different conditions for 21 days. It can be noted that both the positive controls and the peptide fractions prolong the life of the nematodes when compared with the negative NGM control.

## Claims

1. Bioactive product obtained from cocoa extracts, **characterised in that**:
a) it comprises one or more peptides formed by a chain of 5 to 20 amino acids
b) it exerts PEP enzyme inhibitory activity *in vitro.*
c) it has antioxidant and/or antineurodegenerative capacity *in vivo.*
with the exception of bioactive products comprising one or more peptides selected from the group consisting of: SDNEWAWMF (SEQ.ID. No. 29); LSDNEWAWMF (SEQ. ID. No. 30); SDNEWAWMFK (SEQ. ID. No. 31); LSDNEWAWMFK (SEQ. ID. No. 32); RRSDLDNGTPVIF (SEQ. ID. No. 33); DNYDNSAGKWWVT (SEQ. ID. No. 34); TSTVWRLDNYDNSA (SEQ. ID. No. 35) and DNYDNSAGKWWVTTD (SEQ. ID. No. 36).

2. Bioactive product obtained from cocoa extracts according to claim 1, **characterised in that** it comprises one or more peptides from the following group: SEQ. ID. No. 1; SEQ. ID. No. 2; SEQ. ID. No. 3; SEQ. ID. No. 4; SEQ. ID. No. 5; SEQ. ID. No. 6; SEQ. ID. No. 7; SEQ. ID. No. 8; SEQ. ID. No. 9; SEQ. ID. No. 10; SEQ. ID. No. 11; SEQ. ID. No. 12; SEQ. ID. No. 13; SEQ. ID. No. 14; SEQ. ID. No. 15; SEQ. ID. No. 16; SEQ. ID. No. 17; SEQ. ID. No. 18; SEQ. ID. No. 19; SEQ. ID. No. 20; SEQ. ID. No. 21; SEQ. ID. No. 22; SEQ. ID. No. 23; SEQ. ID. No. 24; SEQ. ID. No. 25; SEQ. ID. No. 26; SEQ. ID. No. 27 and SEQ. ID. No. 28.

3. The bioactive product obtained from cocoa extracts according to claims 1-2, **characterised in that** it is obtained by enzymatic hydrolysis from a cocoa product with husk, referred to as *bark.*

4. The bioactive product obtained from cocoa extracts according to claims 1-3, **characterised in that** prior to enzymatic hydrolysis the bark has been defatted by means of physical pressing, until having between 5% and 15% fat, preferably 10%.

5. The bioactive product obtained from cocoa extracts according to claims 1-4 **characterised in that** the defatted product is dissolved in water (5% to 20%) and is subjected to a step of enzymatic hydrolysis, in which one or more enzyme/enzymes is/are added to the aqueous solution obtained and the temperature is maintained at between 40 °C and 60 °C for a time period lasting from 1 to 24 hours, at a suitable pH.

6. The bioactive product obtained from cocoa extracts according to claims 1-5 **characterised in that** enzymatic hydrolysis is performed by one or more enzyme/enzymes belonging to the Termamyl, Alcalase, Neutrase, Ultraflo or Flavourzyme group.

7. The bioactive product obtained from cocoa extracts according to claims 1-6 **characterised in that** the bioactive peptides are obtained by centrifugation, from the supernatant of the hydrolyzed extract, once hydrolysis has finished.

8. The use of the bioactive product according to claims 1-7, comprising one or more peptides from the following group: SEQ. ID. No. 1; SEQ. ID. No. 2; SEQ. ID. No. 3; SEQ. ID. No. 4; SEQ. ID. No. 5; SEQ. ID. No. 6; SEQ. ID. No. 7; SEQ. ID. No. 8; SEQ. ID. No. 9; SEQ. ID. No. 10; SEQ. ID. No. 11; SEQ. ID. No. 12; SEQ. ID. No. 13; SEQ. ID. No. 14; SEQ. ID. No. 15; SEQ. ID. No. 16; SEQ. ID. No. 17; SEQ. ID. No. 18; SEQ. ID. No. 19; SEQ. ID. No. 20; SEQ. ID. No. 21; SEQ. ID. No. 22; SEQ. ID. No. 23; SEQ. ID. No. 24; SEQ. ID. No. 25; SEQ. ID. No. 26; SEQ. ID. No. 27 and SEQ. ID. No. 28, **characterised in that** it is added to functional foods in the amount required to effect antihypertensive and/or antidegenerative and/or antioxidant activity.

9. Functional food according to claim 8, **characterised in that** it comprises one or more bioactive peptides with PEP enzyme inhibitory activity *in vitro* and antioxidant and/or antineurodegenerative capacity *in vivo.*

10. The use of the bioactive product according to claims 1-7 that comprises one or more peptide/peptides from the following group: SEQ. ID. No. 1; SEQ. ID. No. 2; SEQ. ID. No. 3; SEQ. ID. No. 4; SEQ. ID. No. 5; SEQ. ID. No. 6; SEQ. ID. No. 7; SEQ. ID. No. 8; SEQ. ID. No. 9; SEQ. ID. No. 10; SEQ. ID. No. 11; SEQ. ID. No. 12; SEQ. ID. No. 13; SEQ. ID. No. 14; SEQ. ID. No. 15; SEQ. ID. No. 16; SEQ. ID. No. 17; SEQ. ID. No. 18; SEQ. ID. No. 19; SEQ. ID. No. 20; SEQ. ID. No. 21; SEQ. ID. No. 22; SEQ. ID. No. 23; SEQ. ID. No. 24; SEQ. ID. No. 25; SEQ. ID. No. 26; SEQ. ID. No. 27 and SEQ. ID. No 28, for the manufacture of a medicine to treat neurodegenerative conditions.

11. A pharmaceutical composition that comprises one or more peptide/peptides from the following group: SEQ. ID. No. 1, SEQ. ID. No. 2; SEQ. ID. No. 3; SEQ. ID. No. 4; SEQ. ID. No. 5; SEQ. ID. No. 6; SEQ. ID. No. 7; SEQ. ID. No. 8; SEQ. ID. No. 9; SEQ. ID. No. 10; SEQ. ID. No. 11; SEQ. ID. No. 12; SEQ. ID. No. 13; SEQ. ID. No. 14; SEQ. ID. No. 15; SEQ. ID. No. 16; SEQ. ID. No. 17; SEQ. ID. No. 18; SEQ. ID. No. 19; SEQ. ID. No. 20; SEQ. ID. No. 21; SEQ. ID. No. 22; SEQ. ID. No. 23; SEQ. ID. No. 24; SEQ. ID. No. 25; SEQ. ID. No. 26; SEQ. ID. No. 27 and SEQ. ID. No. 28 according to claims 1-7, **characterised in that** said composition has antihypertensive and/or antidegenerative and/or antioxidant activity.


SEQUENCE LISTING

<110>  BIÓPOLIS S.L. y NATRACEUTICAL INDUSTRIAL S.L.U.

<120>  OBTENCIÓN DE PRODUCTOS BIOACTIVOS PROCEDENTES DEL CACAO CON
       ACTIVIDAD INHIBIDORA DE LA ENZIMA PEP Y ACTIVIDAD ANTIOXIDANTE
       Y/O ANTINEURODEGENERATIVA

<130>  AX090268WO

<160>  36

<170>  PatentIn version 3.3

<210>  1
<211>  11
<212>  PRT
<213>  Artificial

<220>
<223>  Biopéptido

<400>  1

Gly Val Lys Gly Glu Pro Gly Pro Asn Thr Leu
1               5                   10


<210>  2
<211>  11
<212>  PRT
<213>  Artificial

<220>
<223>  Biopéptido

<400>  2

Ala Leu Pro Val Asn Ser Pro Gly Lys Tyr Glu
1               5                   10


<210>  3
<211>  13
<212>  PRT
<213>  Artificial

<220>
<223>  Biopéptido

<400>  3

Thr Asp Gly Val Lys Gly Glu Pro Gly Pro Asn Thr Leu
1               5                   10


<210>  4
<211>  12
<212>  PRT
<213>  Artificial

<220>
<223>  Biopéptidos

<400>  4

Leu Ser Gln Ser Pro Val Tyr Ser Asn Gln Asn Gly
1               5                   10

<210>  5
<211>  15
<212>  PRT
<213>  Artificial

<220>
<223>  Biopéptidos

<400>  5

Val Thr Thr Asp Gly Val Lys Gly Glu Pro Gly Pro Asn Thr Leu
1               5                   10                  15


<210>  6
<211>  9
<212>  PRT
<213>  Artificial

<220>
<223>  Biopéptidos

<400>  6

Ser Asp Asp Asp Gly Gln Ile Arg Leu
1               5


<210>  7
<211>  9
<212>  PRT
<213>  Artificial

<220>
<223>  Biopéptidos

<400>  7

Asn Tyr Asp Asn Ser Ala Gly Lys Trp
1               5


<210>  8
<211>  10
<212>  PRT
<213>  Artificial

<220>
<223>  Biopéptidos

<400>  8

Asp Asn Tyr Asp Asn Ser Ala Gly Lys Trp
1               5                   10


<210>  9
<211>  13
<212>  PRT
<213>  Artificial

<220>
<223>  Biopéptidos

<400>  9

Thr Asp Gly Val Lys Gly Glu Pro Gly Pro Asn Thr Leu
1               5                   10

<210>  10
<211>  12
<212>  PRT
<213>  Artificial

<220>
<223>  Biopéptido

<400>  10

Arg Leu Asp Asn Tyr Asp Asn Ser Ala Gly Lys Trp
1               5                   10


<210>  11
<211>  15
<212>  PRT
<213>  Artificial

<220>
<223>  Biopéptido

<400>  11

Val Thr Thr Asp Gly Val Lys Gly Glu Pro Gly Pro Asn Thr Leu
1               5                   10                  15


<210>  12
<211>  18
<212>  PRT
<213>  Artificial

<220>
<223>  Biopéptido

<400>  12

Ala Asn Ser Pro Val Leu Asp Thr Asp Gly Asp Glu Leu Gln Thr Gly
1               5                   10                  15


Val Gln




<210>  13
<211>  7
<212>  PRT
<213>  Artificial

<220>
<223>  Biopéptido

<400>  13

Gly His Ala Val Thr Phe Phe
1               5


<210>  14
<211>  8
<212>  PRT
<213>  Artificial

<220>
<223>  Biopéptido

<400>  14

Phe Ala Ser Lys Asp Gln Pro Leu
1               5

<210>  15
<211>  8
<212>  PRT
<213>  Artificial

<220>
<223>  Biopéptido

<400>  15

Thr Phe Gly Glu Phe Gln Gln Val
1               5

<210>  16
<211>  10
<212>  PRT
<213>  Artificial

<220>
<223>  Biopéptido

<400>  16

Val Ala Pro Ala Gly His Ala Val Thr Phe
1               5                   10

<210>  17
<211>  10
<212>  PRT
<213>  Artificial

<220>
<223>  Biopéptido

<400>  17

Lys Ala Pro Leu Ser Pro Gly Asp Val Phe
1               5                   10

<210>  18
<211>  9
<212>  PRT
<213>  Artificial

<220>
<223>  Biopéptido

<400>  18

Asn Tyr Asp Asn Ser Ala Gly Lys Trp
1               5

<210>  19
<211>  13
<212>  PRT
<213>  Artificial

<220>
<223>  Biopéptido

<400>  19

Ala Pro Leu Ser Pro Gly Asp Val Phe Val Ala Pro Ala
1               5                   10

<210>  20
<211>  12
<212>  PRT
<213>  Artificial

<220>
<223>  Biopéptido

<400>  20

Gln Val Lys Ala Pro Leu Ser Pro Gly Asp Val Phe
1               5                   10

<210>  21
<211>  13
<212>  PRT
<213>  Artificial

<220>
<223>  Biopéptido

<400>  21

Thr Asp Gly Val Lys Gly Glu Pro Gly Pro Asn Thr Leu
1               5                   10

<210>  22
<211>  14
<212>  PRT
<213>  Artificial

<220>
<223>  Biopéptido

<400>  22

Lys Ala Pro Leu Ser Pro Gly Asp Val Phe Val Ala Pro Ala
1               5                   10

<210>  23
<211>  13
<212>  PRT
<213>  Artificial

<220>
<223>  Biopéptido

<400>  23

Gln Gln Val Lys Ala Pro Leu Ser Pro Gly Asp Val Phe
1               5                   10

<210>  24
<211>  16
<212>  PRT
<213>  Artificial

<220>
<223>  Biopéptido

<400>  24

Gln Val Lys Ala Pro Leu Ser Pro Gly Asp Val Phe Val Ala Pro Ala
1               5                   10                  15

<210>  25
<211>  14
<212>  PRT
<213>  Artificial

<220>
<223>  Biopéptido

<400>  25

Ser Gln Ser Pro Val Tyr Ser Asn Gln Asn Gly Arg Phe Phe
1               5                   10

<210>  26
<211>  16
<212>  PRT
<213>  Artificial

<220>
<223>  Biopéptido

<400>  26

Trp Val Thr Thr Asp Gly Val Lys Gly Glu Pro Gly Pro Asn Thr Leu
1               5                   10                  15

<210>  27
<211>  15
<212>  PRT
<213>  Artificial

<220>
<223>  Biopéptido

<400>  27

Ser Gln Ser Pro Val Tyr Ser Asn Gln Asn Gly Arg Phe Phe Glu
1               5                   10                  15

<210>  28
<211>  19
<212>  PRT
<213>  Artificial

<220>
<223>  Biopéptido

<400>  28

Ala Asn Ser Pro Val Leu Asp Thr Asp Gly Asp Glu Leu Gln Thr Gly
1               5                   10                  15

Val Gln Tyr

<210>  29
<211>  9
<212>  PRT
<213>  Artificial

<220>

<223>  Biopéptido descrito en la solicitud de patente PCT/ES2008/000540

<400>  29

Ser Asp Asn Glu Trp Ala Trp Met Phe
1               5


<210>  30
<211>  10
<212>  PRT
<213>  Artificial

<220>
<223>  Biopéptido descrito en la solicitud de patente PCT/ES2008/000540

<400>  30

Leu Ser Asp Asn Glu Trp Ala Trp Met Phe
1               5                   10


<210>  31
<211>  10
<212>  PRT
<213>  Artificial

<220>
<223>  Biopéptido descrito en la solicitud de patente PCT/ES2008/000540

<400>  31

Ser Asp Asn Glu Trp Ala Trp Met Phe Lys
1               5                   10


<210>  32
<211>  11
<212>  PRT
<213>  Artificial

<220>
<223>  Biopéptido descrito en la solicitud de patente PCT/ES2008/000540

<400>  32

Leu Ser Asp Asn Glu Trp Ala Trp Met Phe Lys
1               5                   10


<210>  33
<211>  13
<212>  PRT
<213>  Artificial

<220>
<223>  Biopéptido descrito en la solicitud de patente PCT/ES2008/000540

<400>  33

Arg Arg Ser Asp Leu Asp Asn Gly Thr Pro Val Ile Phe
1               5                   10


<210>  34
<211>  13
<212>  PRT
<213>  Artificial

<220>

<223>  Biopéptido descrito en la solicitud de patente PCT/ES2008/000540

<400>  34

Asp Asn Tyr Asp Asn Ser Ala Gly Lys Trp Trp Val Thr
1               5                   10


<210>  35
<211>  14
<212>  PRT
<213>  Artificial

<220>
<223>  Biopéptido descrito en la solicitud de patente PCT/ES2008/000540

<400>  35

Thr Ser Thr Val Trp Arg Leu Asp Asn Tyr Asp Asn Ser Ala
1               5                   10


<210>  36
<211>  15
<212>  PRT
<213>  Artificial

<220>
<223>  Biopéptido descrito en la solicitud de patente PCT/ES2008/000540

<400>  36

Asp Asn Tyr Asp Asn Ser Ala Gly Lys Trp Trp Val Thr Thr Asp
1               5                   10                  15